# EUROPEAN PATENT APPLICATION

(11) **EP 0 597 340 A1**
(43) Date of publication of application: **18.05.1994**
(21) Application number: 93117598.8
(22) Date of filing: 29.10.1993
(51) Int. Cl.: A61L 15/26, A61L 15/44

(54) **Medication for skin diseases**

(30) Priority: 03.11.1992 IT MI922518
(71) Applicant: S.I.F.RA. SOCIETA ITALIANA FARMACEUTICI RAVIZZA S.p.A., I-37063 Isola Della Scala (IT)
(72) Inventor: Palmieri, Beniamino, I-41100 Modena (IT); Gutierrez, Bernardo, I-21100 Varese (IT)
(74) Representative: Boggio, Luigi

(57) **Abstract**

A medication comprising a panel made from a reticulated silicone mass in which are defined interstices filled with a linear silicone polymer in which at least one drug is dispersed; the panel being produced by: dispersing a drug in a linear silicone polymer; mixing a reticulating silicone mass with silica, additional linear silicone polymer and a platinum catalyst; mixing the product of the two previous stages into a homogeneous dispersion; mixing the homogeneous dispersion with a cross-linking material; and pouring the product into containers.

## Description

The present invention relates to a medication for skin diseases, in particular, for preventing or curing the formation of keloids or other unsightly skin phenomena of closed wounds, such as desquamation, defoliation, hypertrophy, psoriasis, burns and decubitus ulcers.

According to the present state of the art, the above diseases are treated using creams containing various medicinal or medicative substances, including certain vitamins, particularly vitamin E which possesses antioxidizing properties; or using medicated or nonmedicated plasters, or silicone-based medications applied to the skin.

Publication BE O 100 148 relates to a surgical medication comprising a silicone elastomer foam sheet reinforced with fibrous material or cloth of less than 10 mm thickness, and one face of which presents a surface layer of open-cell foam, while the other presents substantially less open cells. The process for producing the medication comprises the application, on an absorbent substrate, of a liquid layer of silicone elastomer capable of producing form, and the addition of a reinforcing material capable of remaining incorporated in the foam.

Publication BE O 300 620 relates to a surgical medication comprising a silicone gel sheet having one surface for application to the wound, possibly with an antiseptic, a bactericide or antifungal agent or similar for curing wounds and burns, or a growth factor, and the other laminated surface of which presents a silicone elastomer film. The process for producing the medication comprises the formation of a cured silicone gel sheet on to which is subsequently laminated a silicone elastomer film.

Publication BE O 401 713 A2 relates to a method of treating skin lesions by applying a dry bandage comprising a flexible thermoplastic film permeable to gas and humidity, and impregnated with a drug. Presumably, the bandage is permeable to water and therefore not washable for reuse.

Creams present the disadvantage of having to be applied, by means of massage, at more or less frequent intervals, and of being of no more than limited effectiveness. A drawback common to all three of the above prior medications, and also of the plaster solution, is that the medication remains active for no more than a few hours, so that numerous successive medications must be applied for any improvement to be achieved. Even the drug-impregnated medication in BE O 300 620 and bandage in BE O 401 713 must be changed frequently for any appreciable improvement to be observed. In fact, by virtue of the silicone gel sheet or thermoplastic film being impregnated with the drug subsequent to formation of the sheet or film, the impregnated drug is released on to the skin as a result of adherence and, preferably, the pressure exerted on the skin. Consequently, for a given type of medication or bandage, the same amount of the drug is transferred to the skin from the impregnated cells, in that part of the drug is absorbed by the skin, while the rest remains inside the cells and is never absorbed however long the medication is applied.

The principal object of the present invention is to provide a medication for effectively treating the above skin diseases, and which provides for overcoming the above drawbacks.

According to the present invention, there is provided a medication for skin diseases, comprising a panel formed from a reticulated silicone mass, the interstices of which are filled with a linear silicone polymer containing at least one drug. As we know, linear silicone polymers have no cross-linking capacity. The reticulated silicone mass is dimethylvinylpolysiloxane-based with a cross-linking agent hydrogenated in the presence of a platinum catylyst, and the linear silicone polymer is dimethylpolysiloxane-based. Tests conducted to determine the behaviour of the panel when applied to the skin have shown the linear silicone polymer in the interstices of the reticulated silicone mass to be in a sort of mechanically unstable conditions, in the sense that it tends to "migrate" from the panel into the skin as soon as the skin is capable of absorbing it. The medication therefore acts as follows: if the skin is dry enough to be absorbent, it draws out and absorbs part of the linear silicone polymer in the panel until it is no longer absorbent. If, at this point, the medication is removed, it may be washed (it is water-repellent) and retained for further application when the skin is once more absorbent. This may be repeated using the same medication for as many as, say, thirty days. If the medication is not removed upon termination of the first migration, it remains inactive until the skin is once more absorbent, at which point, a further portion of the linear silicone polymer again "migrates" into the skin, and so on until the disease is cured or the linear silicone polymer runs out.

Clinical tests have shown a 3 mm thick panel to remain active for roughly 30 days when applied to the skin for roughly 12 hours a day. The above test result is therefore employed to advantage in the process according to the present invention for providing a synergic effect wherein an effective quantity of the linear silicone and a drug is caused to gradually penetrate inside the skin in controlled manner and for a prolonged period of time. What is more, being reusable, the above medication also provides for low-cost treatment. Tests involving treatment of the same skin disease and two groups of ten patients, one treated with a medication containing vitamin E according to the present invention, and the other with a traditional silicone and vitamin E medication, gave satisfactory results in roughly 30 days for the first group as compared with barely acceptable results in 55 to 85 days for the second. The silicone-vitamin E mixture, in fact, has been found to improve vitamin penetration inside the skin, and, by virtue of the vitamin E, inhibit oxidation of the skin cells.

The medication may comprise traditional supporting and fastening means for application to the skin. Generally speaking, the panel will be flat, as suited for application to more or less extensive areas, and of such a thickness as to contain the amount of linear silicone and drug required for treatment. The panel together with the supporting and fastening means will be packed in a conventional container.

The drug may be vitamin E capable of being dispersed in the linear silicone polymer and suitable for the treatment of numerous skin disorders, and may also be any drug which, as such or with the assistance of amphoteric systems, such as "FOMBLIN" (Enimont registered trade mark), can be retained, dispersed or in solution, in the linear silicone polymer and migrate with it into the skin.

"Migration" into the skin of the linear silicone polymer (hereinafter referred to as DMPS) together with the associated drug obviously differs from the "vehicle" or "excipient" function of certain substances contained in creams, ointments or gels, or from the function of certain surface-active agents which, associated with an endermic drug, provide for absorption by modifying the cytoplasmic margins of the keratinocytes.

The advantages of the present invention will be clear from the foregoing description, the foremost of which, however, may be summed up as follows: the silicone together with the drug retained in it penetrates slowly and over a long period of time into the outermost layers of the skin; the medication in itself retains the silicone and the drug for a prolonged period of time, releases both in a number of applications repeated at intervals, and, being water-repellent, may be washed for the purpose of hygiene and retained between one application and the next without the silicone or drug being removed; the medication has a synergic effect, by virtue of the particular capacity of the silicone to penetrate the outermost layers of the skin to which the drug is thus also transferred; penetration of the outermost layers by the silicone is followed by desquamation and, hence, spontaneous removal of the silicone from the skin; and treatment cost is reduced by virtue of the same medication being effectively usable dozens of times.

The process according to the present invention comprises, in succession, the following stages:

### STAGE 1 - silicone/drug mixture

A first mixture is prepared comprising, by weight, 70 to 95% DMPS with a viscosity of 100 to 350 mm²/s; and 30 to 5% vitamin E which is dispersed in the mixture.

### STAGE 2 - reticulated mass mixture

A second mixture is prepared comprising, by weight, 10 to 20% dimethylvinylpolysiloxane; 3 to 10% pyrogenic silica; 60 to 90% DMPS with a viscosity of 1,000 to 10,000 mm²/s as a plasticizer; and, as a catalyst, 10 to 50 mg of organoplatinum compound per Kg of the total mass of the second mixture.

### STAGE 3 - mixing the first and second mixture

1 to 2.5 parts by weight of the first mixture are mixed with 0.5 to 2 parts of the second mixture to produce a homogeneous dispersion of the components.

### STAGE 4 - addition of a cross-linking agent

A solution is prepared comprising, by weight, 60 to 80% DMPS with a viscosity of 1,000 to 10,000 mm²/s as a plasticizer; and 40 to 20% of hydropolymethylhydroxydimethylsiloxane α Ω type cross-linking agent; which solution is added to the homogeneous dispersion obtained in the previous Stage and comprising, by weight, 5 to 15% of the mixture defined in Stage 2.

### STAGE 5 - preparing the panels and package

A number of appropriately flat containers, each with a piece of cloth covering the bottom, are set out, and into each is poured, at room temperature, a controlled quantity of the Stage 4 product, part of which penetrates and adheres inside the interstices in the cloth; the reticulating part of the product reticulates to form in each container a solid panel which, in the interstices of the reticulated portion, retains the DMPS and the vitamin E dispersed in it. This Stage terminates before the vitamin E deteriorates in contact with air. During the cross-linking process, on the surface of the panel in contact with the air, the organoplatinum compound sublimates partially to form a sticky surface, which adheres firmly to, and so provides for occluding and preventing oxidation of, the skin.

The percentages of the components employed in the first four Stages described above may of course vary from one medication to another, depending on the action required and the mechanical characteristics of the medication, migration time and other associated parameters. Moreover, and particularly in Stage 1, substances may be added for improving penetration of the DMPS and vitamin E into the skin, such as a glycol silicone copolymer, or for softening the skin, such as a phenol silicone copolymer, or any other substances capable of enhancing the effectiveness of the medication. Other substances useful in several of the types of therapy under discussion may of course also be dispersed, singly or mixed together, in the linear silicone polymer, in particular, active steroids, cyclosporine for controlling skin transplants, and antibiotic and antifungal substances.

The invention will now be described in more detail with reference to the following example.

### EXAMPLE

### STAGE 1 - silicone/drug mixture

A first mixture is prepared comprising, by weight, 90% linear silicone polymer with a viscosity of 250 mm²/s; and 10% vitamin E which is dispersed in the mixture.

### STAGE 2 - reticulated mass mixture

A second mixture is prepared comprising, by weight, 15% dimethylvinylpolysiloxane; 5% pyrogenic silica; 80% linear silicone polymer with a viscosity of 5,000 mm²/s as a plasticizer; and, as a catalyst, 25 mg of an organoplatinum compound per Kg of the total mass of the second mixture.

### STAGE 3 - mixing the first and second mixtures

2 parts by weight of the first mixture are mixed with 1 part of the second mixture to produce a homogeneous dispersion of the components.

### STAGE 4 - addition of a cross-linking agent

A solution is prepared comprising, by weight, 70% linear silicone polymer with a viscosity of 5,000 mm²/s as a plasticizer; and 30% hydropolymethylhydroxydimethylsiloxane α Ω type cross-linking agent; which solution is added to the homogeneous dispersion obtained in the previous Stage and comprising, by weight, 10% of the mixture defined in Stage 2.

### STAGE 5 - preparing the panels and package

This Stage is the same as Stage 5 described previously.

## Claims

1. A medication for skin diseases, wherein a panel of silicone material retains a drug; characterized by the fact that it comprises a panel made of a reticulated silicone mass in which are defined interstices filled with a linear silicone polymer containing at least one drug.

2. A medication as claimed in Claim 1, characterized by the fact that the reticulated silicone mass is dimethylvinylpolysiloxane-based with a cross-linking agent hydrogenated in the presence of a platinum catalyst; and the linear silicone polymer is dimethylpolysiloxane.

3. A medication as claimed in Claim 1 or 2, characterized by the fact that said drug is vitamin E.

4. A process for producing the medication as claimed in Claims 1, 2 and 3, characterized by the fact that it comprises the following stages: dispersing at least one drug in a linear silicone polymer; mixing a reticulating silicone mass with silica, additional linear silicone polymer and an organoplatinum compound; mixing the products of the two previous stages into a homogeneous dispersion; mixing said homogeneous dispersion with a cross-linking material; and pouring the resulting product into a number of containers, in each of which said reticulating silicone mass reticulates to form a solid panel which retains said linear silicone polymer with said drug dispersed in the interstices of the reticulated portion.

5. A process as claimed in Claim 4, characterized by the fact that:
- in a first stage, a first mixture is prepared comprising, by weight, 70 to 95% of linear silicone polymer with a viscosity of 100 to 350 mm²/s; and 30 to 5% of vitamin E which is dispersed in the mixture;
- in a second stage, a second mixture is prepared comprising, by weight, 10 to 20% of dimethylvinylpolysiloxane; 3 to 10% of pyrogenic silica; 60 to 90% of linear silicone polymer with a viscosity of 1,000 to 10,000 mm²/s as a plasticizer; and, as a catalyst, 10 to 50 mg of an organoplatinum compound per Kg of the total mass of the second mixture;
- in a third stage, 1 to 2.5 parts by weight of said first mixture is mixed with 0.5 to 2 parts of said second mixture to produce a homogeneous dispersion of the components; and
- in a fourth stage, a solution is prepared comprising, by weight, 60 to 80% of linear silicone polymer with a viscosity of 1,000 to 10,000 mm²/s as a plasticizer; and 40 to 20% of hydropolymethylhydroxydimethylsiloxane α Ω type cross-linking agent; which solution is added to the homogeneous dispersion obtained in the previous stage and comprising, by weight, 5 to 15% of the mixture defined in the second stage.

6. A process as claimed in Claim 4, characterized by the fact that:
- in a first stage, a first mixture is prepared comprising, by weight, 90% linear silicone polymer with a viscosity of 250 mm²/s; and 10% vitamin E which is dispersed in the mixture;
- in a second stage, a second mixture is prepared comprising, by weight, 15% dimethylvinylpolysiloxane; 5% pyrogenic silica; 80% linear silicone polymer with a viscosity of 5,000 mm²/s as a plasticizer; and, as a catalyst, 25 mg of an organoplatinum compound per Kg of the total mass of the second mixture;
- in a third stage, 2 parts by weight of said first mixture are mixed with 1 part of said second mixture to produce a homogeneous dispersion of the components; and
- in a fourth stage, a solution is prepared comprising, by weight, 70% of linear silicone polymer with a viscosity of 5,000 mm²/s as a plasticizer; and 30% hydropolymethylhydroxydimethylsiloxane α Ω type cross-linking agent; which solution is added to the homogeneous dispersion obtained in the previous stage and comprising, by weight, 10% of the mixture defined in the second stage.

7. A process as claimed in Claim 5 or 6, characterized by the fact that a fifth stage is performed wherein a number of appropriately flat containers, each covered at the bottom with a porous supporting element, are set up, and into each container is poured, at room temperature, a controlled quantity of the fourth stage product, part of which penetrates and adheres inside the interstices in said porous element; the reticulating part of said product reticulating to form in each container a solid panel which, in the interstices of the reticulated mass, retains the DMPS with the vitamin E dispersed in it; and said stage being concluded before the vitamin E deteriorates in contact with the air.
